# EUROPÄISCHE PATENTANMELDUNG

(11) **EP 1 371 651 A2**
(43) Veröffentlichungstag der Anmeldung: **17.12.2003**
(21) Anmeldenummer: 03012501.7
(22) Anmeldetag: 02.06.2003
(51) Int. Cl.: C07D 277/32

(54) **N-alkylierte Thiazoliumsalze und Verfahren zu deren Herstellung**

(30) Priorität: 10.06.2002 DE 10225537
(71) Anmelder: BAYER AG, 51368 Leverkusen (DE)
(72) Erfinder: Wischnat, Ralf, Dr., 51061 Köln (DE); Rudolph, Joachim, Dr., Guilford, CT 06437 (US)

(57) **Zusammenfassung**

Die vorliegende Erfindung betrifft N-alkylierte Thiazoliumsalze, ein Verfahren zu ihrer Herstellung sowie die Verwendung als Kondensations- und Dehydratisierungsmittel.

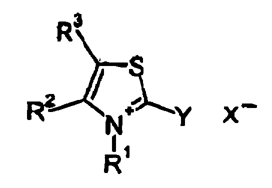

## Beschreibung

Die vorliegende Erfindung betrifft N-alkylierte Thiazoliumsalze, ein Verfahren zu ihrer Herstellung sowie die Verwendung als Kondensations- und Dehydratisierungsmittel.

BEMT, 2-Brom-3-ethyl-4-methyl-thiazolium-tetrafluoroborat, ein N-alkyliertes Thiazoliumsalz, hat Anwendung als Peptidkupplungsreagenz gefunden und wird üblicherweise durch Alkylierung von 2-Brom-4-methyl-thiazol mit Triethyloxonium-tetrafluoroborat, auch Meerweinsalz genannt, hergestellt (siehe P. Li, Tetrahedron Lett. **1999**, *40*, 8301-8304). Das als Ausgangsprodukt verwendete 2-Brom-4-methyl-thiazol wird seinerseits durch eine Sandmeyer-Reaktion ausgehend von 2-Amino-4-methylthiazol erhalten.

Der Nachteil bei dieser Synthesemethode ist jedoch, dass die Darstellung des 2-Brom-4-methyl-thiazols nur mit geringen Ausbeuten möglich ist und die Alkylierung den Einsatz des teuren Triethyloxonium-tetrafluoroborats erfordert.

Es bestand daher das Bedürfnis, ein effizientes Verfahren bereitzustellen, das die N-Alkylierung von Thiazolen in guten Ausbeuten ermöglicht.

Ebenso bestand weiterhin ein Bedarf an weiteren N-alkylierten Thiazoliumsalzen, die mit hoher Effizienz insbesondere als Kondensations- und Dehydratisierungsmittel eingesetzt werden können.

Es wurde nun ein Verfahren zur Herstellung von N-alkylierten Thiazoliumsalzen gefunden, das dadurch gekennzeichnet ist, dass Thiazole der Formel (I) 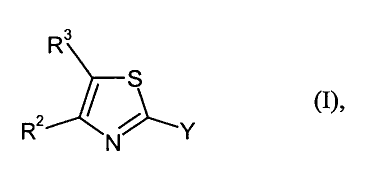 in der
- R²: für Wasserstoff, C₁-C₁₂-Alkyl, C₄-C₁₄-Aryl oder C₅-C₁₅-Arylalkyl und
- R³: für Wasserstoff, C₁-C₁₂-Alkyl, C₄-C₁₄-Aryl oder C₅-C₁₅-Arylalkyl oder
- R² und R³: zusammen für folgende Gruppierungen stehen, 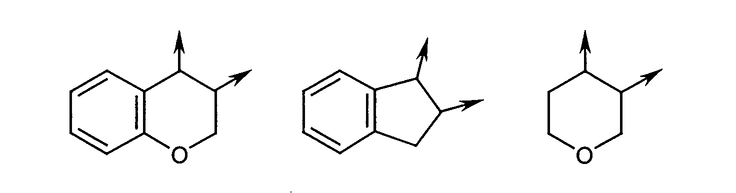 wobei die Pfeile die Verknüpfungspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
- Y: für Iod, Brom oder Chlor steht
in Gegenwart eines Lösungsmittels mit Verbindungen der Formel (II) umgesetzt werden

R¹-X (II)

in der
- R¹: für primäres oder sekundäres C₁-C₃-Alkyl, primäres oder sekundäres C₄-C₁₂-Alkyl oder C₅-C₁₅-Arylalkyl steht und
- X: für Chlor, Brom oder Iod steht
und auf diese Weise Verbindungen der Formel (III) erhalten werden, 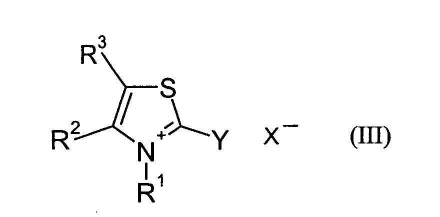 in der
R¹, R² und R³ die unter Formel (I) und X die unter der Formel (II) angegebene Bedeutung besitzen.

Im Rahmen der Erfindung können die oben aufgeführten allgemeinen oder in Vorzugsbereichen aufgeführten Restedefinitionen bzw. Erläuterungen und Parameter untereinander, also auch zwischen den jeweiligen Bereichen und Vorzugsbereichen beliebig kombiniert werden.

Alkyl bzw. Alkoxy steht im Rahmen der Erfindung jeweils unabhängig für einen geradkettigen oder cyclischen, unabhängig davon verzweigten oder unverzweigten Alkyl- bzw. Alkoxy-Rest, die durch C₁-C₄-Alkoxy-Reste weiter substituiert sein können. Gleiches gilt für den Alkylenteil eines Arylalkyl-Restes. Beispielsweise steht im Rahmen der Erfindung C₁-C₃-Alkyl jeweils für Methyl, Ethyl, 2-Ethoxyethyl, n-Propyl und Isopropyl, C₁-C₄-Alkyl darüberhinaus für n-Butyl und tert.-Butyl, C₁-C₆-Alkyl darüber hinaus beispielsweise für n-Pentyl, Cyclohexyl und n-Hexyl, C₁-C₁₂-Alkyl weiter darüber hinaus für n-Heptyl, n-Octyl, iso-Octyl, Norbomyl, n-Decyl und n-Dodecyl. Insbesondere steht primäres oder sekundäres C₄-C₁₂-Alkyl für n-Butyl, sec-Butyl, n-Pentyl, Neopentyl, n-Hexyl, Cyclohexyl, n-Heptyl, n-Octyl, n-Decyl und n-Dodecyl.

Beispielsweise steht C₁-C₄-Alkoxy jeweils für Methoxy, Ethoxy, 2-Ethoxyethoxy, n-Propoxy, Isopropoxy, n-Butoxy und tert.-Butoxy, C₁-C₆-Alkoxy darüber hinaus beispielsweise für n-Pentoxy, Cyclohexoxy und n-Hexoxy.

**Halogenalkyl** bzw. **Halogenalkoxy** steht im Rahmen der Erfindung jeweils unabhängig für einen geradkettigen oder cyclischen, unabhängig davon verzweigten oder unverzweigten Alkyl- bzw. Alkoxy-Rest gemäß obiger Definition, wobei die Reste jeweils einfach, mehrfach oder vollständig durch Halogenatome, vorzugsweise Chlor- und/oder Fluoratome substituiert sind.

Beispielsweise steht C₁-C₆-Halogenalkyl für Trifluormethyl, Trichlormethyl, 2,2,2-Trifluorethyl, Pentafluorethyl und Nonafluorbutyl, C₁-C₆-Halogenalkoxy beispielsweise für Trifluormethoxy, Pentafluorethoxy oder 2,2,2-Trifluorethoxy.

Aryl steht im Rahmen der Erfindung beispielsweise und bevorzugt für carbocyclische aromatische Reste oder heteroaromatische Reste, die keines, ein, zwei oder drei Heteroatome pro Cyclus, im gesamten heteroaromatische Rest mindestens jedoch ein Heteroatom enthalten, das ausgewählt ist aus der Gruppe Stickstoff, Schwefel oder Sauerstoff.

Weiterhin können die carbocyclischen aromatischen Reste oder heteroaromatischen Reste mit einem, zwei oder drei Substituenten pro Cyclus substituiert sein, die jeweils unabhängig voneinander ausgewählt sind aus der Gruppe Nitro, C₁-C₄-Alkylsulfonyl, C₁-C₄-Alkoxycarbonyl, C₁-C₄-Halogenalkoxycarbonyl, C₁-C₄-Alkylcarbonyloxy oder C₁-C₄-Halogenalkylcarbonyloxy, C₁-C₆-Alkyl, Cyano, COO-(C₁-C₁₆-Alkyl), COO-(C₄-C₁₀-Aryl), CO-(C₁-C₆-Alkyl), CO-(C₄-C₁₀-Aryl), O-(C₁-C₆-Alkyl), O-(C₄-C₁₀-Aryl), N(C₁-C₆-Alkyl)₂, Fluor, Chlor, Brom und C₁-C₆-Halogenalkyl. Gleiches gilt für den Arylteil eines Arylalkyl-Restes.

In Formel (III) steht
- R¹: bevorzugt für Methyl, Ethyl, n-Propyl, iso-Propyl, n-Butyl oder für gegebenenfalls einfach oder mehrfach durch Fluor und/oder Chlor, Methyl, Ethyl, n- oder iso-Propyl, Trifluormethyl, Methoxy, Ethoxy substituiertes Benzyl, besonders bevorzugt für Methyl, Ethyl, n-Propyl oder Benzyl und ganz besonders bevorzugt für Methyl, Ethyl und n-Propyl,
- R²: bevorzugt für Wasserstoff, Methyl, Ethyl, n-Propyl, Isopropyl, n-Butyl, sec.- Butyl oder für gegebenenfalls einfach oder mehrfach durch Fluor und/oder Chlor, Methyl, Ethyl, n- oder iso-Propyl, Methoxy, Ethoxy oder n- oder iso- Propoxy substituiertes Benzyl oder Phenyl, besonders bevorzugt für Methyl, Ethyl, n-Propyl, n-Butyl oder für gegebenenfalls einfach oder mehrfach durch Fluor und/oder Chlor, Methyl oder Ethyl substituiertes Phenyl und ganz besonders bevorzugt für Methyl oder Ethyl,
- R³: bevorzugt für Wasserstoff, Methyl, Ethyl oder Isopropyl, besonders bevorzugt für Wasserstoff oder Methyl und ganz besonders bevorzugt für Wasserstoff,
- R² und R³: bevorzugt zusammen für die Gruppierungen 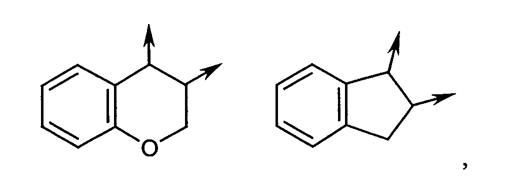 wobei die Pfeile die Verknüpfungspunkte mit dem Thiazolring kennzeichnen oder zusammen für 1,3-Propandiyl oder 1,4-Butandiyl,
- Y: bevorzugt für Iod oder Brom, besonders bevorzugt für Brom,
- X⁻: bevorzugt für Bromid und Iodid, besonders bevorzugt für Bromid.

Für das erfindungsgemäße Verfahren bevorzugte Verbindungen der Formel (I) sind: 2-Chlor-4-methyl-thiazol, 2-Brom-4-methyl-thiazol, 2-Jod-4-methyl-thiazol, 2-Chlor-thiazol, 2-Brom-thiazol, 2-Jod-thiazol, 2-Chlor-4,5-dimethyl-thiazol, 2-Brom-4,5-dimethyl-thiazol, 2-Jod-4,5-dimethyl-thiazol, 2-Chlor-4-ethyl,5-methyl-thiazol, 2-Brom-4-ethyl-5-methyl-thiazol und 2-Jod-4-ethyl-5-methyl-thiazol.

Bevorzugte Verbindungen der Formel (II) sind:
Methylchlorid, Methylbromid, Methyliodid, Ethylchlorid, Ethylbromid, Ethyliodid Benzylchlorid, Benzylbromid und Benzyliodid.

Das erfindungsgemäße Verfahren wird in Gegenwart von Lösungsmittel durchgeführt. Als Lösungsmittel geeignet sind beispielsweise gegebenenfalls halogenierte aliphatische und aromatische Kohlenwasserstoffe wie beispielsweise Petrolether, Hexan, Heptan, Cyclohexan, Methylcyclohexan, Benzol, Toluol, Xylol oder Decalin, Chlorbenzol, Dichlorbenzol, Methylenchlorid, Chloroform, Tetrachlormethan, Dichloroder Trichlorethan oder Tetrachlorethylen, amidische Lösungsmittel wie beispielsweise Dimethylacetamid oder Dimethylformamid, Sulfoxide und Sulfone wie beispielsweise Dimethylsulfoxid oder Tetramethylensulfon oder Mischungen aus den genannten Lösungsmitteln.

Bevorzugt sind Dimethylformamid und Dimethylacetamid.

In einer bevorzugten Ausführungsform des erfmdungsgemäßen Verfahrens können beispielsweise pro mol der Verbindung der Formel (I) 1,0 bis 10,0 mol, bevorzugt 1,1 bis 10,0 mol, besonders bevorzugt 2,1 bis 10,0 mol und ganz besonders bevorzugt 2,5 bis 5 mol Verbindung der Formel (II) eingesetzt werden.

Die Konzentration der Verbindung der Formel (II) im Lösungsmittel kann beispielsweise und bevorzugt 0,2 bis 6,0 mol/L betragen, besonders bevorzugt 2,5 bis 6,0 mol/L.

Die Zugabe der Verbindung der Formel (II) kann direkt zu Reaktionsbeginn oder kontinuierlich gegebenenfalls portionsweise erfolgen.

Die Reaktionstemperatur kann beispielsweise 20 bis 100°C betragen bevorzugt sind 50 bis 75°C. Besonders bevorzugt wird innerhalb von 1 bis 6 Stunden bevorzugt 1,5 bis 2,5 Stunden langsam auf 50 bis 75°C aufgeheizt.

Als Reaktionsgefäß können gewöhnliche Glasapparaturen dienen. Es kann sich allerdings vorteilhaft auswirken, wenn bedingt durch die Siedetemperatur der Reagenzien oder des Lösungsmittels Drücke entstehen und daher in druckbeständigen Gefäßen gearbeitet wird. Als Druckgefäße sind beispielsweise inerte Stahlgefäße oder Gefäße aus Stahllegierungen (beispielsweise Hastelloy, VA-Stahl oder Alloy) geeignet.

Der Reaktionsdruck kann beispielsweise 1 bis 150 bar betragen, bevorzugt sind 3 bis 10 bar, besonders bevorzugt 3 bis 6 bar.

Die Reaktionszeit richtet sich nach der Reaktivität des verwendeten Verbindung der Formel (II) und kann beispielsweise 1 bis 100 Stunden betragen, bevorzugt 1 bis 48 Stunden. Ganz besonders bevorzugt sind Reaktionszeiten von 11 bis 18 h.

Insbesondere bei Verbindungen der Formel (II), die einen Siedepunkt besitzen der bei Normaldruck 50°C oder weniger beträgt, ist es vorteilhaft die Reaktion unter den oben genannten Vorzugsbereichen des Drucks durchzuführen und mit fortschreitender Reaktion den Druckverlust durch Aufpressen eines Inertgases wie beispielsweise Sauerstoff, Stickstoff, Luft oder Argon auszugleichen. Eine gute Durchmischung kann sich ebenfalls vorteilhaft auf die Reaktion auswirken. Bevorzugt wird die Mischung gerührt oder geschüttelt. Besonders bevorzugt sind Durchmischungen die beispielsweise durch die Verwendung von Blattrührern, Intermikrührern oder Ankerrührern herbeigeführt werden.

Die Verbindungen der Formel (III) können gegebenenfalls nach Abkühlen auf Raumtemperatur durch Kristallisation, Filtration oder Extraktion isoliert werden. Bevorzugt ist die Isolierung durch Filtration. Reste und Verunreinigungen können durch Waschen des kristallinen Produktes beispielsweise mit einem inerten Lösungsmittel beispielsweise einem Kohlenwasserstoff oder Halogenkohlenwasserstoff entfernt werden. Die Produkte werden vorteilhafterweise unter Feuchtigkeitsausschluss gelagert.

Ein weiterer Aspekt der Erfindung betrifft die Umsetzung von Verbindungen der Formel (III) mit Halogeniden der Formel (IV)

M(Y²)ₙ (IV)

in der
- M: für ein Metallion mit der Wertigkeit n oder ein quarternäres Ammoniumion wie zum Beispiel Tetrabutylammonium steht und
- Y²: für Chlor, Brom oder Iod steht, wobei Verbindungen
der Formel (IIIb) erhalten werden 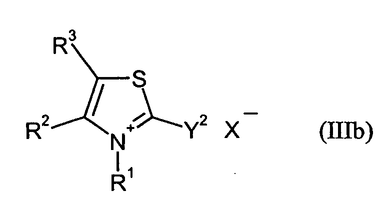 in der
- R¹, R², R³ und X⁻: die unter der Formel (III) und
- Y²: die unter Formel (IV) genannten Bedeutungen besitzen.

Das Verfahren eignet sich insbesondere um Verbindungen der Formel (III) in denen Y für Chlor oder Brom steht in Verbindungen der Formel (IIIb) zu überführen in der Y² für Iod steht, oder um Verbindungen der Formel (III) in denen Y für Chlor steht in Verbindungen der Formel (IIIb) zu überführen in der Y² für Brom oder Iod steht.

Als Verbindungen der Formel (IV) sind bevorzugt Alkali-, Erdalkali- und Übergangsmetallchloride, -bromide und -iodide geeignet, besonders bevorzugt sind Lithium-, Natrium-, Kalium-, Magnesium-, Calcium-, Eisen(III)-, Zink(II)-, Kupfer(II)-chlorid, -bromid und -iodid, wobei die genannten Bromide und Iodide noch weiter bevorzugt sind.

Ein weiterer Aspekt der Erfindung betrifft die Herstellung von Verbindungen der Formel (IIIc) 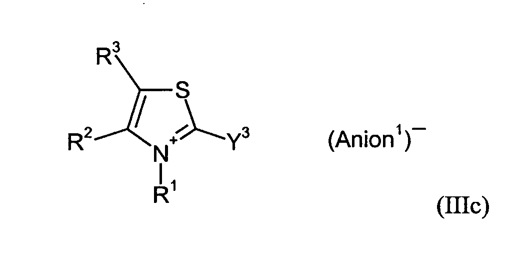 in der
- R¹, R² und R³: die unter der Formel (III) genannte Bedeutung besitzen und
- Y³: für Chlor, Brom oder Iod steht und
(Anion¹)⁻ für ein Anion einer anorganischen Säure oder einer Sulfonsäure steht, wobei Halogenide ausgenommen sind, durch Anionenaustausch.

Bevorzugt steht (Anion¹)⁻ für Tetrafluoroborat, Hexafluorophosphat, ein halbes Äquivalent Sulfat und Sulfononate des Typs R⁴SO₃⁻, wobei R⁴ für C₁-C₆-Alkyl, C₁-C₆-Halogenalkyl oder C₄-C₁₀-Aryl gemäß obiger Definition steht.

Besonders bevorzugt steht (Anion¹)⁻ für Tetrafluoroborat und Hexafluorophosphat, wobei Tetrafluoroborat noch weiter bevorzugt ist.

Der Anionenaustausch kann vorteilhafterweise in der Art erfolgen, dass ein Anionentauscher mit Säuren des Typs H(Anion¹) beladen wird, wobei (Anion¹) die oben genannte Bedeutung einschließlich der genannten Vorzugsbereiche besitzt und mit den Verbindungen der Formeln (III) oder (IIIb) zur Reaktion gebracht wird.

Von der Erfindung sind auch die bisher nicht bekannten Thiazoliumsalze der Formel (IIId) umfasst, 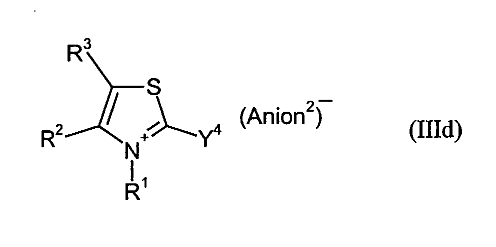 in der
- R² und R³: die unter der Formel (I) genannte Bedeutung und Vorzugsbereiche besitzen und
- Y⁴: für Chlor, Brom oder Iod steht und
für den Fall dass
- Y⁴: für Chlor oder Brom steht
- R¹: für primäres oder sekundäres C₄-C₁₂-Alkyl steht und
für den Fall dass
- Y⁴: für Iod steht
- R¹: für primäres oder sekundäres C₁-C₃-Alkyl, primäres oder sekundäres C₄-C₁₂-Alkyl oder C₅-C₁₅-Arylalkyl steht,
und weiterhin für R¹ ansonsten die gleichen Vorzugsbereiche gelten, die unter der Formel (II) genannt wurden
und
- (Anion²)⁻: für ein Anion einer anorganischen Säure oder einer Sulfonsäure steht. Bevorzugt steht (Anion²)⁻ für Chlorid, Bromid, Iodid, Tetrafluoroborat, Hexafluorophosphat, ein halbes Äquivalent Sulfat oder Sulfonate des Typs R⁴SO₃⁻, wobei R⁴ für C₁-C₆-Alkyl, C₁-C₆-Halogenalkyl oder C₄-C₁₀-Aryl gemäß obiger Definition steht, besonders bevorzugt für Chlorid, Bromid, Iodid, Tetrafluoroborat und Hexafluorophosphat.

Als Verbindungen der Formel (IIId) seien genannt:
2-Chlor-3,4-dimethyl-thiazoliumchlorid, 2-Brom-3,4-dimethyl-thiazoliumbromid, 2-Jod-3,4-dimethyl-thiazoliumjodid, 2-Chlor-3-methyl-thiazoliumchlorid, 2-Brom-3-methyl-thiazoliumbromid, 2-Jod-3-methyl-thiazoliumjodid, 2-Chlor-3,4,5-trimethylthiazoliumchlorid, 2-Brom-3,4,5-trimethyl-thiazoliumbromid, 2-Jod-3,4,5-trimethylthiazoliumjodid, 2-Chlor-4-ethyl, 3,5-dimethyl-thiazoliumchlorid, 2-Brom-4-ethyl-3,5-dimethyl-thiazoliumbromid und 2-Jod-4-ethyl-3,5-dimethyl-thiazoliumjodid.

Die als Edukte für die Herstellung von Verbindungen der Formel (III) verwendeten Verbindungen der Formel (I) lassen sich besonders vorteilhaft durch Cyclisierung von alpha-Thiocyanatoketonen herstellen. Daher ist von der Erfindung auch ein Verfahren umfasst, dass dadurch gekennzeichnet ist, dass die Verbindungen der Formel (I) 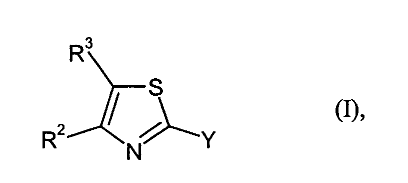 durch Umsetzung von Verbindungen der Formel (V), 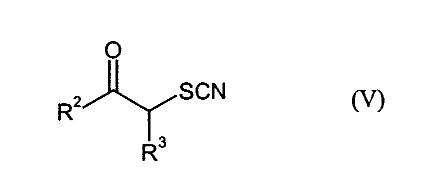 in der
R² und R³ die gleiche Bedeutung und Vorzugsbereiche besitzen, die unter der Formel (I) oben genannt wurden
mit Säuren H(Anion³) hergestellt werden und in Gegenwart von Lösungsmittel zu Thiazoliumsalzen der Formel (VI) umgesetzt werden 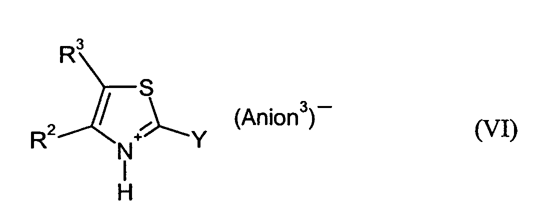 in der (Anion³)⁻ für ein Halogenid steht und die Verbindungen der Formel (VI) in einem weiteren Schritt durch Umsetzung mit einer Base in Verbindungen der Formel (I) überführt werden.

Beispielsweise sind als Säuren H(Anion³) geeignet: Chlorwasserstoff, Iodwasserstoff und Bromwasserstoff.

Die als Ausgangsprodukte verwendeten Verbindungen der Formel (IV) sind bekannt und/oder können nach bekannten Verfahren oder analog dazu hergestellt werden (siehe z. B. Schantl et al. 1998, Synth. Commun. 28, 1451-1462, Indian J. Chem., Sect. B (1991), 30, 1152-1155).

Von der Erfindung sind weiterhin die bislang nicht bekannten Verbindungen der Formel (VIb) umfasst 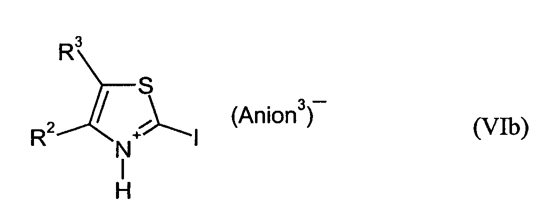 in der
R² und R³ die gleiche Bedeutung und Vorzugsbereiche besitzen die unter der Formel (I) genannt wurden und
- (Anion³)⁻: die gleiche Bedeutung und Vorzugsbereiche besitzt die oben genannt wurden.

Als Einzelverbindungen der Formel (VIb) seien genannt:
2-Jod-3,4-dimethyl-thiazoliumjodid, 2-Jod-3-methylthiazoliumjodid, 2-Jod-3,5-dimethyl-thiazoliumjodid und 2-Jod-4-ethyl-3-methyl-thiazoliumjodid.

Als Lösungsmittel für die Cyclisierung seien beispielsweise genannt: Aliphatische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Methylenchlorid, Chloroform, Tetrachlormethan, Dichlor-, Trichlorethan oder Tetrachlorethylen; Ether, wie Diethyl-, Diisopropyl-, Methyl-t-butyl-, Methyl-t-Amylether, Dioxan, Tetrahydrofuran, 1,2-Dimethoxyethan, 1,2-Diethoxyethan, Diethylenglykoldimethylether oder Anisol. Bevorzugt setzt man Methylenchlorid, Chloroform, 1,2-Dichlorethan, Diethyloder tert.-Butylmethylether, besonders bevorzugt Methylenchlorid ein.

Die Menge des Lösungsmittels pro mol Verbindung der Formel (V) kann beispielsweise 0,5 bis 5 l, bevorzugt 1 bis 3 l betragen.

Man kann bei der Cyclisierung pro Mol einer Verbindung der Formel (V) 2,0 bis 10,0 mol, bevorzugt 2,1 bis 7 mol Säure. Die Angaben für Halogenid gelten analog, jedoch unabhängig von der Säuremenge.

Die Cyclisierung wird vorteilhafterweise unter Feuchtigkeitsausschluss durchgeführt. Dieses kann man beispielsweise dadurch gewährleisten, dass man handelsübliche trockene Verdünnungsmittel einsetzt oder diese nach den allgemein üblichen Trocknungsmethoden trocknet, weiterhin trockene Säuren einsetzt. Beispielsweise kann Bromwasserstoff bzw. Chlorwasserstoff durch eine tiefgekühlte Gasfalle und/oder einen Trockenturm mit einem geeigneten Trockenmittel oder eine Gaswascheinrichtung, wie z.B. eine Waschflasche mit konzentrierter Schwefelsäure, geleitet werden. Die Cyclisierung führt man zweckmäßigerweise so durch, dass man die Verbindung der Formel (IV) vorzugsweise in dem Verdünnungsmittel vorlegt und dann den Halogenwasserstoff einleitet oder das Halogenid und dann die Säure unter Temperaturkontrolle und guter Verteilung zugibt. Die exotherme Reaktion führt man im Allgemeinen bei einer Temperatur von -30 bis +40°C, bevorzugt bei -15 bis +30°C durch.

Die daraus resultierenden Verbindungen der Formel (VI) kann dann bequem durch ein Fest-Flüssig-Abtrennverfahren, wie beispielsweise Abfiltrieren oder Zentrifügieren, erhalten werden.

Zur Freisetzung des Verbindungen der Formel (I) sind organische und anorganische Basen geeignet. Hierzu gehören vorzugsweise Alkalimetallcarbonate oder -hydrogencarbonate, wie beispielsweise Natrium-, Kalium- oder Ammoniumcarbonat, Natriumhydrogen- oder Kaliumhydrogencarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen. Bevorzugt sind Natriumhydrogen- oder Kaliumhydrogencarbonat.

Die Freisetzung der Verbindungen der Formel (I) aus den Verbindungen der Formel (VI) kann vorteilhafterweise ohne vorherigen Trocknungsschritt in dem gleichen Verdünnungsmittel wie die Cyclisierung erfolgen. Hierzu ist es vorteilhaft, beim Abtrennen der Verbindung der Formel (VI) mit etwas Verdünnungsmittel nachzuwaschen, um gegebenenfalls vorhandene Säure weitgehend zu entfernen. Beispielsweise suspendiert man die Verbindung der Formel (VI) im Verdünnungsmittel und gibt dann die Base hinzu. Bevorzugt setzt man eine wässrige Lösung einer anorganischen Base ein, wie beispielsweise Natriumhydrogencarbonatlösung. Die Konzentration ist dabei unkritisch. Vorzugsweise nimmt man höherkonzentrierte bis gesättigte Lösungen. Pro mol der Verbindung (V) setzt man beispielsweise 1,0 bis 1,5 mol, bevorzugt 1,0 bis 1,2 mol Base ein. Die Neutralisation führt man im Allgemeinen bei einer Temperatur von -20 bis +30°C, bevorzugt bei -5 bis +10°C durch.

Die Isolierung des Verbindungen der Formel (I) erfolgt nach den üblichen Methoden der organischen Chemie. Vorzugsweise führt man eine Phasentrennung durch und destilliert die organische Phase. Man kann vor der Destillation eine Trocknung mit einem Trockenmittel wie beispielsweise Magnesium- oder Natriumsulfat, Calciumchlorid, Silicagel oder Molekularsieb durchführen.

Weiterhin sind von der Erfindung die bislang unbekannten Thiazole der Formel (Ib) umfasst, 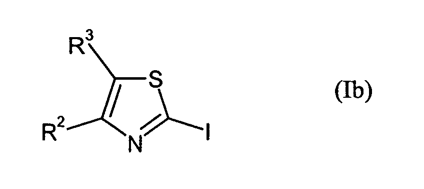 in der
R² und R³ die gleiche Bedeutung und Vorzugsbereiche besitzen die unter der Formel (I) genannt wurden.

Die Verbindungen der Formel (IIId) sowie die ebenfalls erfindungsgemäß herstellbaren Verbindungen der Formeln (III), (IIIb) und (IIIc) eignen sich insbesondere für die Anwendung in Kondensations- und Dehydratisierungsreaktionen sowie für Halogenierungen.

Im Rahmen der Erfindung sind unter Kondensationsreaktionen sind solche Reaktionen zu verstehen, bei denen unter formaler Abspaltung von Wasser die Neuausbildung einer chemischen Bindung zwischen zwei Funktionalitäten erfolgt. Bevorzugte Beispiele für Kondensationsreaktionen sind Verfahren zur Herstellung von amidischen Bindungen aus Carbonsäuren und Aminen einschließlich der Herstellung von Peptiden, die gegebenenfalls cyclisch sein können, aus Aminosäuren sowie Verfahren zur Herstellung von Lactamen aus Aminocarbonsäuren, Verfahren zur Herstellung von Estern aus Carbonsäuren und Alkoholen einschließlich der Herstellung von Lactonen aus Hydroxycarbonsäuren, Verfahren zur Herstellung von Anhydriden aus zwei gleichen oder verschiedenen Carbonsäuren einschließlich der Herstellung von cyclischen Anhydriden aus Dicarbonsäuren, Verfahren zur Herstellung von Isoxazolen aus Oximen von Hydroxyketonen oder Benzoxazinonen aus Oximen von Acylphenolen und Verfahren zur Herstellung von Oxazolinen aus N-(beta-hydroxyalkyl)carbonsäureamiden oder Thiazolinen aus N-(beta-hydroxyalkyl)-thiocarbon-säureamiden.

Im Rahmen der Erfindung sind unter Dehydratisierungsreaktionen sind solche Reaktionen zu verstehen in denen unter formaler Abspaltung von Wasser der Bindungsgrad einer bereits vorhandenen chemischen Bindung erhöht wird. Beispiele sind das Verfahren zur Herstellung von Nitriloxiden aus primären Nitroalkylverbindungen, Verfahren zur Herstellung von Alkenen aus Alkoholen wie insbesondere die Herstellung von α-Ketoolefinen aus α-Hydroxyketonen oder β-Hydroxyketonen, Verfahren zur Herstellung von Nitrilen aus Aldoximen oder Carbonsäureamiden, Verfahren zur Herstellung von Carbodiimiden aus Harnstoffen und Verfahren zur Herstellung von Isonitrilen aus Formylamiden.

Im Rahmen der Erfindung sind unter Halogenierungen sind solche Reaktionen zu verstehen in denen eine Hydroxylgruppe in eine Halogenfünktion überführt wird. Insbesondere umfassen Halogenierungen Verfahren zur Herstellung von Alkylfluoriden, -chloriden, -bromiden und -iodiden aus Alkoholen in Gegenwart des entsprechenden Halogenids.

Weitere Details gehen aus den nachfolgenden Beispielen hervor, ohne dass sich dadurch die Erfindung darauf beschränkt.

### Herstellungsbeispiele

### Beispiel 1

### Synthese von 2-Brom-3,4-Dialkyl-thiazoliumbromid

100 g (0,56 mol) 2-Brom-4-methylthiazol werden in 106 ml wasserfreiem Dimethylformamid gelöst und die Mischung bei Raumtemperatur in einen HC-Autoklaven eingetragen. Nun werden 213,28 g (2,25 mol) Brommethan aufgepresst, so das der Druck auf 1,9 bar ansteigt. Anschließend wird das Reaktionsgemisch bei guter Durchmischung mit einer Heizrate von 8°C/Minute auf 70°C erwärmt und die Temperatur solange isobar gehalten, bis die Umsetzung möglichst quantitativ ist. Die Umsetzung wird dabei massenspektroskopisch verfolgt. Zur Aufarbeitung kühlt man auf Raumtemperatur, entspannt den Autoklaven und filtriert das Produkt ab. Lösungsmittelreste und Verunreinigungen werden durch Waschen mit 300 ml Hexan entfernt und das Produkt im Hochvakuum getrocknet.
Ausbeute: 107,5 g (70 % d. Theorie)
Schmp.: 221°C

### Beispiel 2

### Synthese von 2-Iod-3,4-Dimethyl-thiazoliumjodid

0,55 g (2,0 mmol) 2-Brom-3,4-dimethylthiazoliumbromid (siehe auch Beispiel 1) werden in 10 ml Dimethylformamid gelöst und bei Raumtemperatur mit 0,33 g (2,0 mmol) Kaliumjodid versetzt. Zur vollständigen Umsetzung wird für 5 h auf 50°C erwärmt. Der Umsatz wird dabei über EI oder FD MS-Spektroskopie verfolgt. Nach vollständiger Umsetzung kann 2-Iod-3,4-dimethyl-thiazoliumjodid für die beschriebenen Umsetzungen eingesetzt werden.
Ausbeute: quantitativ (100 % d. Theorie)
Analytik: MS

### Beispiel 3

### Synthese von N-Acetylanilin

0,3 g (0,01 mol) Essigsäure und 1,36 g (0,01 mol) 2-Brom-3,4-dimethylthiazoliumbromid werden bei -5°C in 20 ml Dichlormethan gelöst und mit 1,29 g (0,01 mol) N,N-Diisopropylethylamin versetzt. Man rührt 30 min nach und versetzt die Mischung bei 0°C nochmals mit 1,29 g (0,01 mol) N,N-Diisopropylethylamin und 0,47 g (0,01 mol) Anilin. Der Reaktionsverlauf wird per Dünnschichtchromatographie verfolgt. Zur Aufarbeitung verdünnt man mit 20 ml Dichlormethan und wäscht die organische Phase nacheinander mit gesättigter Ammoniumchloridlösung, 1N Salzsäure und gesättigter Natriumchloridlösung. Die organische Phase wird über Magnesiumsulfat getrocknet und das Lösungsmittel abdestilliert. Der Rückstand wird an Kieselgel chromatographiert.
Ausbeute: 0,39 g (58 % d. Theorie), weiße Kristalle
Schmp.: 113-114°C

### Beispiel 4

### Synthese von 2-Acetyl-amino-5-bromothiazol-4-carbonsäureethylester

0,3 g (0,01 mol) Essigsäure werden bei Raumtemperatur in 10 ml Dichlormethan gelöst und mit 1,29 g (0,01 mol) Diisopropyl-ethylamin versetzt. Zu dieser Lösung gibt man 1,36 g (0,01 mol) 2-Brom-3,4-dimethylthiazoliumbromid und 1,29 g (0,01 mol) Diisopropylethylamin. Man rührt für 10 min. nach und versetzt die Mischung mit 1,26 g (0,01 mol) 2-Amino-5-bromthiazol-4-carbonsäureethylester. Zur Aufarbeitung verdünnt man mit 20 ml Dichlormethan und wäscht die organische Phase mit 1 N HCl, und gesättigter Natriumchloridlösung. Die organische Phase wird über MgSO₄ getrocknet und das Lösungsmittel am Rotationsverdampfer abdestilliert. Der Rückstand wird an Kieselgel chromatographiert mit Hexan/Essigsäureethylester im Gradient 2:1 nach 1:1.
Ausbeute: 270 mg (19 %)
Schmp: 201-203°C

### Beispiel 5

### Synthese von Benzoesäureanhydrid

0,61 g (5 mmol) Benzoesäure in 10 ml Dichlormethan werden bei 0°C mit 1,29 g (10 mmol) Diisopropylethylamin versetzt, gefolgt von 1,37 g (5 mmol) 2-Brom-3,4-dimethylthiazoliumbromid. Der Reaktionsverlauf wird per Dünnschichtchromatographie verfolgt. Zur Aufarbeitung verdünnt man mit 20 ml Dichlormethan und wäscht die organische Phase nacheinander mit 1 N HCl und gesättigter Natriumchloridlösung. Das Lösungsmittel wird über MgSO₄ getrocknet und nach Filtration abdestilliert. Zur Aufreinigung filtriert man über Kieselgel mit Dichlormethan als Eluens. Zur Isolierung des reinen Produktes wird das Lösungsmittel abdestilliert.
Ausbeute: 0,36 g (64 % d. Theorie)
GC (MS/CI): cal. M/Z= 226.23, gef.: M/Z= 226

### Beispiel 6

### Synthese von Z-Phg-Ala-OMe

1,5 g (5,26 mmol) Z-(S)-Phenylglycin werden bei - 5°C in 10 ml abs. Dichlormethan gelöst und mit 1,36 g (10,52 mmol) Ethyldiisopropylamin versetzt, gefolgt von 1,44g (5,26 mmol) 2-Brom-3,4-dimethylthiazoliumbromid. Man rührt bei 0°C 30 min nach und versetzt mit einer Mischung aus 0,73 g (5,26 mmol) L-Alaninmethylesterhydrochlorid und 0,68 g (5,26 mmol) Ethyldiisopropylamin in 10 ml Dichlormethan. Man rührt für 2 h bei 0°C nach und versetzt zur Aufarbeitung mit 20 ml Dichlormethan und wäscht die organische Phase nacheinander mit gesättigter Ammoniumchloridlösung und gesättigter Natriumchloridlösung. Die organische Phase wird über MgSO4 getrocknet und das Lösungs-mittel am Rotationsverdampfer abdestilliert. Der Rückstand wird an Kieselgel mit Essigsäureethylester/Hexan 3:7 filtriert.
Ausbeute: 0,6 g (31 %)
1H-NMR (400 MHz, CDCl₃): 1.42 (d, 3H, CH₃-Ala, J= 7.1 Hz), 3.72 (s, 3H, OCH₃), 4.55 (m, 1H, CHCH₃), 5.01 - 5.3 (m, br, 3H), 6.01 (s, br, NH), 6.3 (m, br, NH). 7.1 - 7.6 (m, 10H, Aromat).

## Patentansprüche

1. Verfahren zur Herstellung von N-alkylierten Thiazoliumsalzen, **dadurch gekennzeichnet, dass** Thiazole der Formel (I) 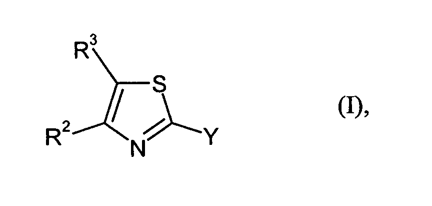 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl
oder
R² und R³ zusammen für folgende Gruppierungen stehen, 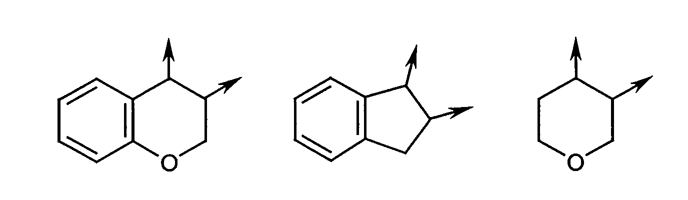 wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
Y für Iod, Brom oder Chlor steht
in Gegenwart eines Lösungsmittels mit Verbindungen der Formel (II) umgesetzt werden
R¹-X (II)
in der
R¹ für primäres oder sekundäres C₁-C₃-Alkyl, primäres oder sekundäres C₄-C₁₂-Alkyl oder C₅-C₁₅-Arylalkyl steht
X für Chlor, Brom oder Iod steht
und auf diese Weise Verbindungen der Formel (III) erhalten werden, 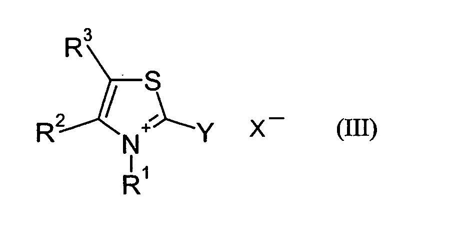 in der
R¹, R² und R³ die unter Formel (I) und X die unter der Formel (II) angegebene Bedeutung besitzen.

2. Verfahren nach Anspruch 1, **dadurch gekennzeichnet, dass**
R¹ für Methyl, Ethyl, n-Propyl, iso-Propyl, n-Butyl oder für gegebenenfalls einfach oder mehrfach durch Fluor und/oder Chlor, Methyl, Ethyl, n- oder iso-Propyl, Trifluormethyl, Methoxy, Ethoxy substituiertes Benzyl steht.

3. Verfahren nach einem oder mehreren der Ansprüche 1 bis 2, **dadurch gekennzeichnet, dass**
R² für Methyl, Ethyl, n-Propyl, Isopropyl, n-Butyl, sec.-Butyl oder für gegebenenfalls einfach oder mehrfach durch Fluor und/oder Chlor, Methyl, Ethyl, n- oder iso-Propyl, Methoxy, Ethoxy oder n- oder iso-Propoxy substituiertes Benzyl oder Phenyl steht.

4. Verfahren nach einem oder mehreren der Ansprüche 1 bis 3, **dadurch gekennzeichnet, dass**
R³ für Wasserstoff, Methyl, Ethyl oder Isopropyl steht.

5. Verfahren nach einem oder mehreren der Ansprüche 1 bis 2, **dadurch gekennzeichnet, dass**
R² und R³ zusammen für die Gruppierungen 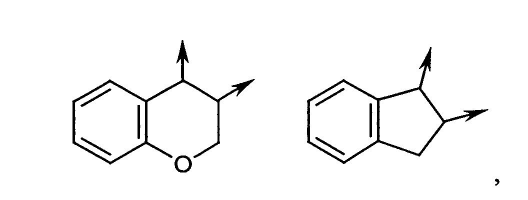 wobei die Pfeile die Verknüpfungspunkte mit dem Thiazolring kennzeichnen oder zusammen für 1,3-Propandiyl oder 1,4-Butandiyl stehen.

6. Verfahren nach einem oder mehreren der Ansprüche 1 bis 5, **dadurch gekennzeichnet, dass** als Verbindungen der Formel (I) 2-Chlor-4-methylthiazol, 2-Brom-4-methyl-thiazol, 2-Jod-4-methyl-thiazol, 2-Chlor-thiazol, 2-Brom-thiazol, 2-Jod-thiazol, 2-Chlor-4,5-dimethyl-thiazol, 2-Brom-4,5-dimethyl-thiazol, 2-Jod-4,5-dimethyl-thiazol, 2-Chlor-4-ethyl,5-methylthiazol, 2-Brom-4-ethyl-5-methyl-thiazol und 2-Jod-4-ethyl-5-methyl-thiazol eingesetzt werden.

7. Verfahren nach einem oder mehreren der Ansprüche 1 bis 6, **dadurch gekennzeichnet, dass** als Verbindungen der Formel (II) Methylchlorid, Methylbromid, Methyliodid, Ethylchlorid, Ethylbromid, Ethyliodid, Benzylchlorid, Benzylbromid oder Benzyliodid eingesetzt werden.

8. Verfahren nach einem oder mehreren der Ansprüche 1 bis 7, **dadurch gekennzeichnet, dass** als Lösungsmittel gegebenenfalls halogenierte aliphatische und aromatische Kohlenwasserstoffe, amidische Lösungsmittel, Sulfoxide und Sulfone oder Mischungen aus den genannten Lösungsmitteln eingesetzt werden.

9. Verfahren nach einem oder mehreren der Ansprüche 1 bis 8, **dadurch gekennzeichnet, dass** der Reaktionsdruck 1 bis 150 bar beträgt.

10. Verfahren zur Herstellung von Verbindungen der Formel (IIIb) 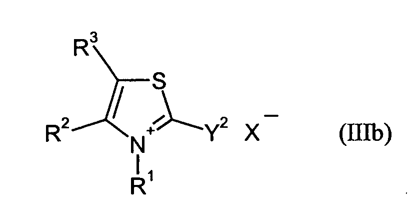 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl oder
R² und R³ zusammen für folgende Gruppierungen stehen, 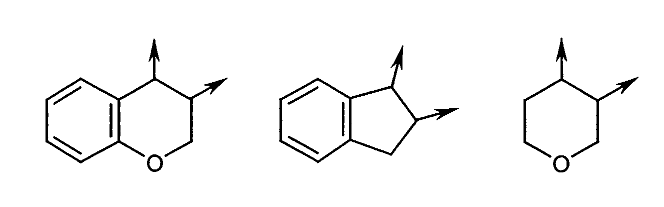 wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
Y² für Chlor, Brom oder Iod steht und
X für Chlor, Brom oder Iod steht
**dadurch gekennzeichnet, dass** Verbindungen der Formel (III) 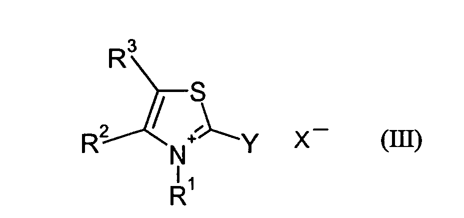 in der
R¹, R² und R³ die unter Formel (I) und X die unter der Formel (In angegebene Bedeutung besitzen,
mit Halogeniden der Formel (IV) umgesetzt werden
M(Y²)ₙ (IV)
in der
M für ein Metallion mit der Wertigkeit n oder ein tertiäres Ammoniumion steht und
Y² die unter der Formel (IIIb) genannte Bedeutung besitzt.

11. Verfahren nach Anspruch 10, **dadurch gekennzeichnet, dass** als Halogenide der Formel (IV) Alkali-, Erdalkali- und Übergangsmetallchloride, -bromide oder -iodide eingesetzt werden.

12. Verfahren zur Herstellung von Verbindungen der Formel (IIIc) 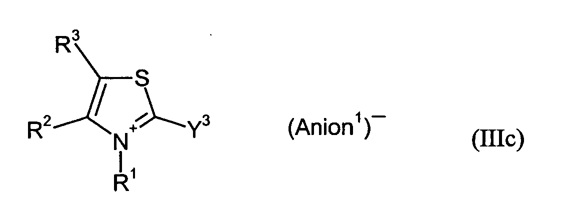 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl oder
R² und R³ zusammen für folgende Gruppierungen stehen, 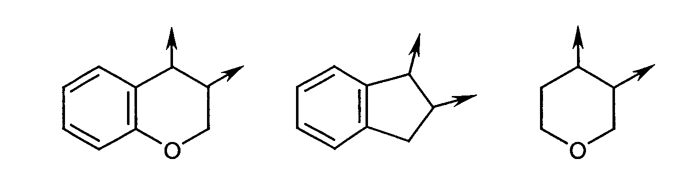
wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
Y³ für Chlor, Brom oder Iod steht und
(Anion¹)⁻ für ein Anion einer Säure oder einer Sulfonsäure steht, **dadurch gekennzeichnet dass** ein Anionenaustausch durchgeführt wird.

13. Verfahren nach Anspruch 12, **dadurch gekennzeichnet, dass** der Anionenaustausch in der Art erfolgt, dass ein Anionentauscher mit Säuren des Typs H(Anion¹) beladen wird, wobei (Anion¹) die in Anspruch 12 genannte Bedeutung besitzt und mit Verbindungen der Formeln (III) oder (IIIb) zur Reaktion gebracht wird, wobei die Verbindungen der Formeln (III) oder (IIIb) die in den Ansprüchen 1 bzw. 10 genannten Bedeutungen besitzen.

14. Thiazoliumsalze der Formel (IIId), 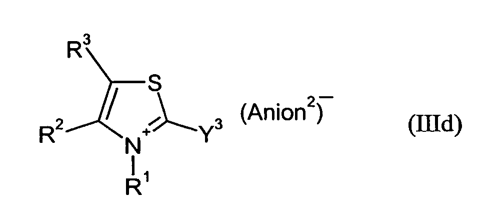 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl oder
R² und R³ zusammen für folgende Gruppierungen stehen, 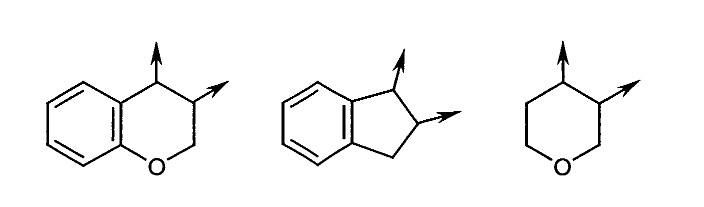
wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
Y³ für Chlor, Brom oder Iod steht und
für den Fall dass
Y³ für Chlor oder Brom steht
R¹ für primäres oder sekundäres C₄-C₁₂-Alkyl steht und
für den Fall dass
Y³ für Iod steht
R¹ für primäres oder sekundäres C₁-C₃-Alkyl, primäres oder sekundäres C₄-C₁₂-Alkyl oder C₅-C₁₅-Arylalkyl steht und
(Anion²)⁻ für ein Anion einer anorganischen Säure oder einer Sulfonsäure steht.

15. 2-Chlor-3,4-dimethyl-thiazoliumchlorid, 2-Brom-3,4-dimethyl-thiazoliumbromid, 2-Jod-3,4-dimethyl-thiazoliumjodid, 2-Chlor-3-methyl-thiazoliumchlorid, 2-Brom-3-methyl-thiazoliumbromid, 2-Jod-3-methyl-thiazoliumjodid, 2-Chlor-3,4,5-trimethyl-thiazoliumchlorid, 2-Brom-3,4,5-trimethylthiazoliumbromid, 2-Jod-3,4,5-trimethyl-thiazoliumjodid, 2-Chlor-4-ethyl, 3,5-dimethyl-thiazoliumchlorid, 2-Brom-4-ethyl-3,5-dimethyl-thiazoliumbromid und 2-Jod-4-ethyl-3,5-dimethyl-thiazoliumjodid.

16. Verfahren nach einem oder mehreren der Ansprüche 1 bis 9, **dadurch gekennzeichnet, dass** die als Edukte verwendeten Verbindungen der Formel (I) durch Cyclisierung von alpha-Thiocyanatoketonen hergestellt werden.

17. Verfahren nach Anspruch 16, **dadurch gekennzeichnet, dass** die Verbindungen der Formel (I) durch Umsetzung von Verbindungen der Formel (V), 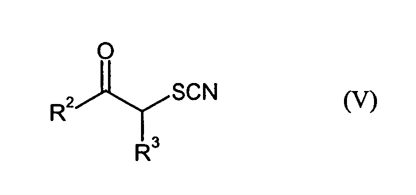 in der
R² und R³ die gleiche Bedeutung besitzen, die unter der Formel (I) im Anspruch 1 genannt wurden
mit Chlorwasserstoff, Bromwasserstoff oder Iodwasserstoff hergestellt werden und in Gegenwart von Lösungsmittel zu Thiazoliumsalzen der Formel (VI) umgesetzt werden 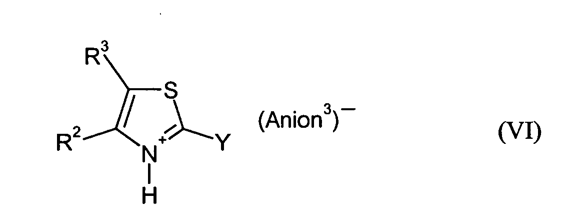 in der (Anion³)⁻ für ein Halogenid steht und die Verbindungen der Formel (VI) in einem weiteren Schritt durch Umsetzung mit einer Base in Verbindungen der Formel (I) überrührt werden.

18. Verbindungen der Formel (VIb) 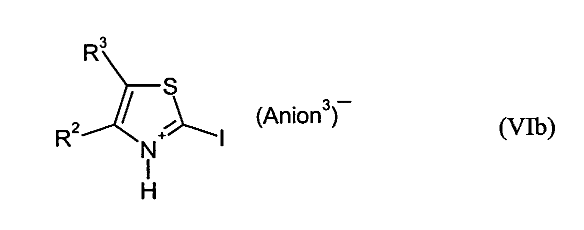 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl oder
R² und R³ zusammen für folgende Gruppierungen stehen, 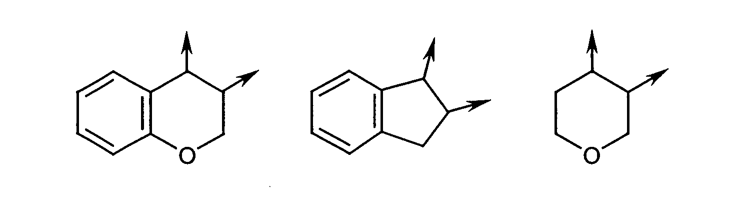
wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können und
(Anion³)⁻ die gleiche Bedeutung und Vorzugsbereiche besitzt die oben genannt wurde.

19. 2-Jod-3,4-dimethyl-thiazoliumjodid, 2-Jod-3-methylthiazoliumjodid, 2-Jod-3,5-dimethyl-thiazoliumjodid und 2-Jod-4-ethyl-3-methyl-thiazoliumjodid.

20. Thiazole der Formel (Ib), 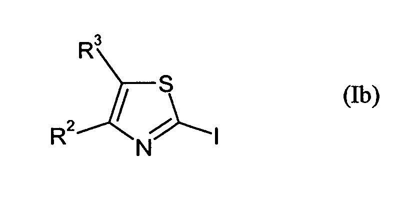 in der
R² für Wasserstoff, C₁-C₁₂-Alkyl, C₄-C₁₄-Aryl oder C₅-C₁₅-Arylalkyl und
R³ für Wasserstoff, C₁-C₁₂-Alkyl, C₄-C₁₄-Aryl oder C₅-C₁₅-Arylalkyl oder
R² und R³ zusammen für folgende Gruppierungen stehen, 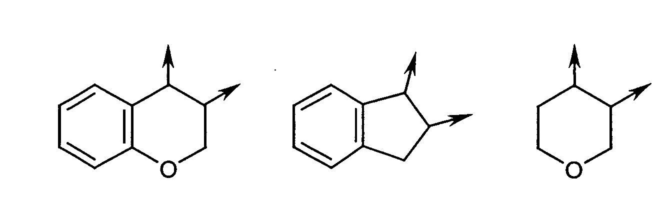
wobei die Pfeile die Verknüpfüngspunkte mit dem Thiazolring kennzeichnen oder R² und R³ zusammen für Reste stehen, die ausgewählt sind aus der Gruppe 1,3-Propandiyl, 1,4-Butandiyl oder 1,5-Pentandiyl, wobei die genannten Gruppierungen und Reste weiterhin einfach oder mehrfach durch Halogen, Nitro, Formyl, C₁-C₆-Alkyl, C₁-C₆-Halogenalkyl, C₁-C₆-Alkoxy oder C₁-C₆-Halogenalkoxy substituiert sein können.

21. Verwendung von Verbindungen gemäß einem oder mehreren der Ansprüche 14 und 15 oder den gemäß einem oder mehreren der Ansprüche 1 bis 13 hergestellten Verbindungen in Kondensations- und Dehydratisierungsreaktionen sowie für Halogenierungen.

22. Verwendung gemäß Anspruch 21, **dadurch gekennzeichnet, dass** Kondensationsreaktionen Verfahren zur Herstellung von amidischen Bindungen aus Carbonsäuren und Aminen einschließlich der Herstellung von Peptiden, die gegebenenfalls cyclisch sein können, aus Aminosäuren sowie Verfahren zur Herstellung von Lactamen aus Aminocarbonsäuren, Verfahren zur Herstellung von Estern aus Carbonsäuren und Alkoholen einschließlich der Herstellung von Lactonen aus Hydroxycarbonsäuren, Verfahren zur Herstellung von Anhydriden aus zwei gleichen oder verschiedenen Carbonsäuren einschließlich der Herstellung von cyclischen Anhydriden aus Dicarbonsäuren, Verfahren zur Herstellung von Isoxazolen aus Oximen von Hydroxyketonen oder Benzoxazinonen aus Oximen von Acylphenolen und Verfahren zur Herstellung von Oxazolinen aus N-(beta-hydroxyalkyl)carbonsäureamiden oder Thiazolinen aus N-(beta-hydroxyalkyl)-thiocarbon-säureamiden sind.

23. Verwendung gemäß Anspruch 21, **dadurch gekennzeichnet, dass** Dehydratisierungsreaktionen Verfahren zur Herstellung von Nitriloxiden aus primären Nitroalkylverbindungen, Verfahren zur Herstellung von Alkenen aus Alkoholen, Verfahren zur Herstellung von Nitrilen aus Aldoximen oder Carbonsäureamiden, Verfahren zur Herstellung von Carbodiimiden aus Harnstoffen und Verfahren zur Herstellung von Isonitrilen aus Formylamiden sind.

24. Verwendung nach Anspruch 21, **dadurch gekennzeichnet, dass** Halogenierungen Verfahren zur Herstellung von Alkylfluoriden, -chloriden, -bromiden oder -iodiden aus Alkoholen sind.
